# EUROPÄISCHE PATENTSCHRIFT

(11) **EP 2 769 714 B1**
(45) Veröffentlichungstag und Bekanntmachung des Hinweises auf die Patenterteilung: **06.05.2015**
(21) Anmeldenummer: 14169050.3
(22) Anmeldetag: 07.11.2007
(51) Int. Cl.: A61K 9/70, A61K 31/485

(54) **Transdermales therapeutisches System zur Verabreichung des Wirkstoffs Buprenorphin**
Transdermal therapeutic system for administering the active substance buprenorphine
Système thérapeutique transdermique pour l'administration de la substance actif buprénorphine

(30) Priorität: 21.11.2006 DE 102006054731
(43) Veröffentlichungstag der Anmeldung: 27.08.2014
(62) Teilanmeldung aus: 07846537.4
(73) Patentinhaber: LTS LOHMANN Therapie-Systeme AG, 56626 Andernach (DE)
(72) Erfinder: Hille, Thomas, 56567 Neuwied (DE); Horstmann, Michael, 56564 Neuwied (DE); Müller, Walter, 56626 Andernach (DE)
(74) Vertreter: Ehlich, Eva Susanne

(56) Entgegenhaltungen:
- EP-A- 0 430 019
- WO-A-98/36728
- WO-A-2004/014336
- DE-A1-102004 062 614
- US-A- 5 240 711
- US-A1- 2001 002 259

## Beschreibung

Die vorliegende Erfindung betrifft ein transdermales therapeutisches System mit mindestens einer, die Löslichkeit des Buprenorphins in der Matrixschicht bestimmenden und ebenfalls resorbierbaren Carbonsäure zur Schmerztherapie mit deutlich erhöhter Wirkstoffausnutzung.

Der Wirkstoff Buprenorphin (17-(Cyclopropylmethyl)-α-(1,1-dimethylethyl)-4,5-epoxy-18,19-dihydro-3-hydroxy-6-methoxy-α-methyl-6,14-ethenomorphinan-7-methanol) ist ein partial synthetisches Opiat, dessen Vorteil gegenüber anderen Verbindungen dieser Substanzklasse in einer höheren Wirksamkeit liegt. Dies bedeutet, dass Schmerzfreiheit bei Krebs - oder Tumorpatienten mit infauster Diagnose im Finalstadium - mit Tagesdosen um 1 mg erreicht werden kann. Dabei zeichnet sich Buprenorphin gegenüber dem synthetischen Opioid Fentanyl und seinen Analogen dadurch aus, dass sein Suchtpotential geringer ist als das dieser Verbindungen. Der Nachteil besteht darin, dass aufgrund des hohen Molekulargewichtes von Buprenorphin, nämlich 467,64 Dalton, es nur schwer zur transdermalen Resorption zu bringen ist.

Trotzdem sind bereits buprenorphinhaltige transdermale Systeme (z. B. Transtec® oder Norspan®) im Handel erhältlich. In dem deutschen Patent DE 39 39 376 C1 ist deren Funktionsweise beschrieben. Der Wirkstoff ist in einer Polyacrylatmatrix homogen gelöst, wobei eine Carbonsäure als Permeationsenhancer und Löslichkeitsvermittler dient.

Systeme, in denen der Wirkstoff homogen gelöst vorliegt, zeichnen sich gewöhnlich durch eine niedrige Wirkstoffausnutzung aus. Der Grund dafür ist, dass die die Wirkstoffabgabe bestimmende thermodynamische Aktivität des Wirkstoffs während der Applikation durch die sinkende Wirkstoffbeladung abnimmt. Eine über die gesamte Applikationszeit gleichmäßige Wirkstoffabgabe ist nur durch eine im Vergleich zur abzugebenden Menge relativ hohe Wirkstoffbeladung zu erreichen. Aus den veröffentlichten Daten zu dem in Europa vermarkteten Produkt Transtec® 35 lässt sich z.B. eine Wirkstoffausnutzung von lediglich 17 % über den Anwendungszeitraum herleiten. Da Buprenorphin ein teurer Wirkstoff ist, wäre eine höhere Wirkstoffausnutzung unter Kostenaspekten von wesentlichem Vorteil.

Eine möglichst geringe Beladung des Systems mit dem zu den Betäubungsmitteln gehörenden Buprenorphin und ein daraus resultierender möglichst geringer Restgehalt nach Anwendung in den benutzten Systemen ist zudem unter Sicherheitsaspekten sehr erstrebenswert.

Die Aufgabe der bestehenden Erfindung war es deshalb, ein TTS zu entwickeln, das den transdermal nur schwer zur Resorption zu bringenden Wirkstoff Buprenorphin mit deutlich erhöhter Wirkstoffausnutzung der transdermalen Applikation zugänglich macht.

Diese Aufgabe wird erfindungsgemäß in überraschender Weise durch ein Transdermales therapeutisches System zur Verabreichung von Buprenorphin an die Haut gelöst, wobei das TTS eine wirkstoffundurchlässige Rückschicht, mindestens eine haftklebende, den Wirkstoff Buprenorphin und mindestens eine Carbonsäure enthaltende Matrixschicht und gegebenenfalls eine vor Gebrauch ablösbare Schutzschicht umfasst. Die Matrixschicht ist auf Basis von Polysiloxanen oder Polyisobutylen aufgebaut. Das Buprenorphin ist in der Carbonsäure oder den Carbonsäuren gelöst und diese Lösung in Form von Tröpfchen in der Matrixschicht dispergiert. Dies ist umso erstaunlicher, als Buprenorphin aufgrund seiner bekannten physikochemischen Eigenschaften, insbesondere durch seine schlechte Löslichkeit, seines vergleichsweise hohen Schmelzpunktes von 216 °C und seines bereits erwähnten hohen Molekulargewichtes, leicht zur Kristallisation neigt. Aus diesem Grunde wird ein Lösemittel mit mindestens einer sauren Gruppe eingesetzt, um die Kristallisation von Buprenorphin während der Lagerung der Arzneiform zu verhindern. Sowohl Buprenorphin selbst als auch Carbonsäuren haben in Polysiloxanen oder Polyisobutylen eine äußerst geringe Löslichkeit. Es ist dadurch möglich, Buprenorphin in einer Carbonsäure zu lösen und diese Lösung in der Form von Tröpfchen in einer auf Basis von Polysiloxanen, bevorzugt aminresistenten Dimethylpolysiloxanen, besonders bevorzugt einem Gemisch aus einem aminresistenten und einem nicht-aminresistenten Dimethylpolysiloxan, wobei das nicht-aminresistente Dimethylpolysiloxan zu maximal 40 Gew.-%, bevorzugt 2 bis 20 Gew.-%, enthalten ist, oder Polyisobutylen als Basispolymer hergestellten Matrixschicht zu dispergieren. Dabei ist wichtig, dass die Mischung aus Buprenorphin und Carbonsäure oder Carbonsäuren flüssig vorliegt.

Üblicherweise sind die eingesetzten Carbonsäuren in den organischen Lösemitteln der Kleber schwerlöslich. Deshalb kann die flüssige Mischung aus Buprenorphin und Carbonsäure in der Lösung des Klebers dispergiert werden, wobei die Dispersion nach Entfernen des Lösemittels erhalten bleibt. Die Löslichkeit des Buprenorphins hängt in einer solchen Matrixschicht praktisch nur von der Menge der Carbonsäure oder Carbonsäuren ab. Die Menge der dispergierten Lösung kann bis zu 40 Gew.-% betragen, wobei bevorzugt 20 Gew.-% nicht überschritten werden. Die Tröpfchengröße selbst sollte vorzugsweise 50 µm nicht überschreiten. Die bevorzugte Größe hängt darüber hinaus von der Dicke der Matrixschicht ab.

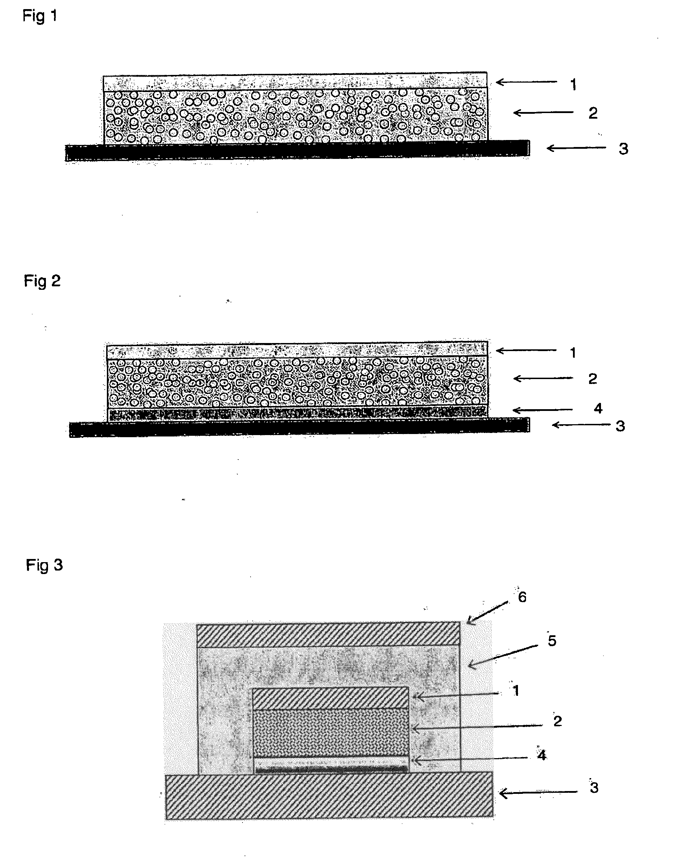

Figur 1 zeigt eine schematische Darstellung eines solch einschichtigen selbstklebenden Systems. In Figur 2 ist ein System mit Hautkontaktschicht dargestellt, Figur 3 zeigt ein mehrschichtiges System mit Überpflaster. Darin sind:
1 Rückschicht
2 Matrixschicht auf Basis von Polysiloxanen mit dispergierten Tröpfchen einer Buprenorphin-Carbonsäurelösung
3 vor Gebrauch zu entfernende Schutzschicht
4 Hautkontaktschicht auf Basis eines Polyacrylatklebers
5 Haftklebschicht ohne Buprenorphin
6 Rückschicht (z.B. hautfarben)

Da Carbonsäuren ebenfalls durch die Haut resorbiert werden können, erniedrigt sich während der Applikationszeit ihre Menge im System und damit auch die Sättigungslöslichkeit des Buprenorphins. Dadurch wird die durch die Abgabe verursachte Abnahme der thermodynamischen Aktivität des Buprenorphins kompensiert. Die Wahl der Carbonsäure richtet sich nach der Resorption durch die Haut, die, verglichen mit Buprenorphin, genauso schnell, bevorzugt schneller, ist. Bevorzugt werden bei Hauttemperatur flüssige Carbonsäuren eingesetzt. Die Carbonsäure oder die Carbonsäuren ist oder sind ausgewählt aus der Gruppe bestehend aus Ölsäure, Lävulinsäure Linolsäure, und Linolensäure. Bei geeigneter Ausführung gelingt es, während der Applikationszeit übersättigte Zustände zu erreichen. In übersättigten Systemen ist die thermodynamische Aktivität des Wirkstoffs und damit auch die Permeationsrate pro Flächeneinheit entsprechend dem Übersättigungsfaktor erhöht. Vorteilshafterweise kann dadurch die Abgabefläche und auch die Fläche des Systems minimiert werden. Während der Lagerung verbleiben sowohl Buprenorphin als auch die Säure in der Polymermatrix, sodass das System während dieser Zeit höchstens gesättigt ist und eine Rekristallisation des Wirkstoffs ausgeschlossen ist.

Ein weiterer Aspekt der Erfindung betrifft den Effekt, dass der Anstieg der thermodynamische Aktivität in solchen Systemen bei zu schneller Abgabe der Säure dazu führen kann, dass die Permeationsrate nach Applikation zu stark ansteigt. Die Folge ist, dass sich das TTS durch eine zu schnelle Wirkstoffabgabe vorzeitig erschöpft. Es wurde nun gefunden, dass ein solcher Effekt durch Hinzufügen einer weiteren Schicht auf Basis von Polyacrylaten verhindert wird. Diese Schicht befindet sich bevorzugt zwischen wirkstoffhaltiger Polymermatrixschicht und Haut oder aber zwischen Matrix- und Rückschicht. Diese zusätzliche Schicht wird bevorzugt als selbstklebende Hautkontaktschicht ausgeführt.

Die Löslichkeit des Buprenorphins in Polyacrylaten ist deutlich höher als in Polysiloxanen oder Polyisobutylen und erreicht, in Abhängigkeit von der genauen Zusammensetzung, bis zu etwa 10 Gewichtsprozente. Da das Gesamtsystem dadurch eine höhere Sättigungslöslichkeit für Buprenorphin hat, wird der durch die Abgabe der Säure verursachte Grad der Übersättigung durch Umverteilung des Buprenorphins von der Matrix- in die Polyacrylatschicht reduziert. Die Wirkstoffabgabe gestaltet sich dadurch gleichmäßiger und eine vorzeitige Erschöpfung des Systems wird verhindert. Es hat sich gezeigt, dass in einer bevorzugten Ausführungsform bei einer Beladung der Matrixschicht mit etwa 0,4 mg Buprenorphin und der Verwendung der Carbonsäure Lävulinsäure, pro cm² eine Hautkontaktschicht mit einem Beschichtungsgewicht von 15 - 30 g/m² ausreichend ist, um den gewünschten Effekt zu erreichen.

Hinsichtlich der für die Herstellung des Polyacrylatklebers verwendeten Monomere gibt es keine grundsätzlichen Einschränkungen. Ausgehend von theoretischen Betrachtungen sind allerdings Kleber ohne freie Carboxylgruppen bevorzugt, da diese das basische Buprenorphin über Salzbildung nicht immobilisieren können.

Figur 2 zeigt eine schematische Darstellung eines solchen Systems; die Herstellung ist in Beispiel 1 beschrieben. Die Dicke der Matrix- und Hautkontaktschicht muss jeweils in Abhängigkeit von der gewählten Wirstoffkonzentration in der Matrixschicht bzw. der Wirkstoffmenge pro Flächeneinheit optimiert werden. Die Menge bzw. die Konzentration der Säure in der Matrixschicht hängt von deren Löslichkeitsvermögen für Buprenorphin ab. Bei der bevorzugten Verwendung der Lävulinsäure werden Buprenorphin und die Säure in gleichen Gewichtsverhältnissen eingesetzt. Die gewählte Konzentration beider Substanzen von 7 bis 9 Gew.-% hat sich als geeignet erwiesen, kann aber unter entsprechender Berücksichtigung bei der Wahl des Beschichtungsgewichts ohne Auswirkung auf die Leistung des TTS auch anders gewählt werden.

Transdermale therapeutische Systeme gemäß Beispiel 1 wurden in einer Pharmakokinetikstudie am Menschen mit bereits vermarkteten TTS als Referenzsystem verglichen. Es zeigte sich, dass ein 17 cm² großes System gemäß Beispiel 1 mit einem Buprenorphingehalt von 6,3 mg einem 25 cm² großen Referenz-TTS mit einem Wirkstoffgehalt von 20 mg entspricht. Unter Berücksichtigung der deklarierten Abgabe des Referenzprodukts von 35 µg/h TTS ergibt sich damit für das Referenzprodukt eine Wirkstoffausnutzung von 17 % und für ein TTS gemäß Beispiel 1 eine Wirkstoffausnutzung von 53 %. Dies zeigt deutlich, dass mit transdermalen Systemen gemäß Beispiel 1 das Ziel einer wesentlich verbesserten Wirkstoffausnutzung erreicht wurde. Mit dem erfindungsgemäßen TTS mit Buprenorphin als Wirkstoff lassen sich somit in vivo Wirkstoffausnutzungen von mindestens 30 %, bevorzugt mindestens 40 %, besonders bevorzugt mindestens 50 % erzielen. Zudem ergibt sich der Vorteil, dass diese Systeme wegen der höheren Permeationsrate flächenmäßig um etwa 30 % kleiner als die Referenzsysteme eingesetzt werden können.

Von besonderem Vorteil ist, dass durch diese verbesserte Wirkstoffausnutzung die Beladung des Systems mit dem zu den Betäubungsmitteln gehörenden Buprenorphins weiter verringert werden kann und somit der in den gebrauchten Systemen verbleibende Restgehalt an Buprenorphin nach Anwendung weiter minimiert ist.

Die erfindungsgemäßen transdermalen therapeutischen Systeme können mit unterschiedlichen Freisetzungsprofilen und in unterschiedlichen Dosisstärken bereitgestellt werden. Wie bereits oben beschrieben, kann z. B. durch entsprechende Variation der Schichtdicke der wirkstoffhaltigen Matrix und/oder der Hautkontaktschicht oder durch Veränderung der Wirkstoffkonzentration in der Matrix Einfluss auf das Wirkstofffreisetzungsprofil genommen werden. Die Dosisstärke des erfindungsgemäßen TTS kann z. B. dadurch angepasst werden, dass bei gleicher Zusammensetzung und Schichtdicke der Matrix und Hautkontaktschicht die Fläche der wirkstoffhaltigen Matrix variiert wird, um so zu verschiedenen Dosisstärken zu gelangen. Vorzugsweise können so transdermale therapeutische Systeme erhalten werden, die vergleichbare Eigenschaften aufweisen, wie die bereits am Markt befindlichen transdermalen therapeutischen Systeme.

Durch die Bereitstellung unterschiedlich stark dosierter TTS ist es möglich, einen Patienten individuell auf die für ihn notwendige Wirkstoffmenge einzustellen. Darüber hinaus wird es möglich, die Wirkstoffabgabe an den Patienten so einzurichten, dass er auf prinzipiell bekannte Weise durch ein entsprechendes Dosierungsschema auf die für ihn erforderliche Wirkstoffmenge titriert wird. Dabei wird die dem Patienten verabreichte Wirkstoffmenge beispielsweise durch sequentielle Verabreichung von transdermalen therapeutischen Systemen mit verschiedener Dosisstärke entsprechend gesteigert. Durch die sequentielle Steigerung der Wirkstoffdosis können die für den Wirkstoff Buprenorphin bekannten Nebenwirkungen, die gegebenenfalls bei dessen Verabreichung auftreten können, weiter reduziert werden. Beispiele für die sequentielle Anpassung der Wirkstoffabgabe an einen Patienten durch entsprechende Dosierschemata sind z. B. in den Patentanmeldungen WO 2006/030030 A2 und EP 1 572 167 beschrieben. Somit umfasst die vorliegende Erfindung auch Systeme, z. B. Kits, die mehrere erfindungsgemäße TTS mit unterschiedlichen Dosisstärken umfassen.

Die erfindungsgemäßen transdermalen therapeutischen Systeme können derart ausgestaltet sein, dass sie ein Unterteilen des TTS in verschiedene Untereinheiten ermöglichen. Durch eine solche Teilbarkeit wird ebenfalls eine weitere Anpassung des TTS an den individuellen Wirkstoffbedarf eines Patienten bzw. die Verwendung des TTS zur Implementierung eines entsprechenden Dosierschemata ermöglicht. Vorteilhafterweise liegen dabei in dem teilbaren TTS eine Vielzahl von Polymermatrixbereichen vor, die räumlich durch wirkstofffreie Bereiche getrennt sind. Die Teilung des TTS kann dann entlang der wirkstofffreien Bereiche erfolgen, beispielsweise durch Schneiden, so dass ein oder mehrere Polymermatrixbereiche von dem Rest des TTS abgetrennt werden. Beispiele für den Aufbau teilbarer TTS-Varianten sind z. B. in den Patentanmeldungen WO 2003/079962 A2 und WO 02/41878 A2 beschrieben.

Die erfindungsgemäßen transdermalen therapeutischen Systeme können für unterschiedliche Applikationszeiträume verwendet bzw. angepasst werden. Die erfindungsgemäßen TTS können beispielsweise jeweils für mindestens 12 h oder 24 h appliziert werden. Vorzugsweise können die einzelnen erfindungsgemäßen TTS aber auch über einen jeweiligen Applikationszeitraum von mindestens 72 h, 84 h oder 96 h verwendet werden. Längere Applikationszeiträume sind aber auch möglich, wie beispielsweise 120 h, 144 h oder 168 h.

Die Erfindung wird durch die folgenden Beispiele näher erläutert, ohne damit aber den Erfindungsumfang einzuschränken:

### Beispiel 1

A In einem Edelstahlbehälter suspendiert man 3,65 Kg Buprenorphin in 3,65 Kg Lävulinsäure und 2,6 Kg Ethanol. Unter Rühren werden 60,6 Kg eines Polysiloxanklebers als Lösung in n-Heptan mit einem Feststoffgehalt von 74 Gew.-% und 9,72 Kg Heptan zugefügt. Man rührt bis zur vollständigen Auflösung der Buprenorphin-Base und erhält 80,22 Kg einer buprenorphinhaltigen Kleberlösung mit 4,55 % Buprenorphin bei einem Feststoffgehalt von 64,8 % (Kleberlösung 1).
B Für die Hautkontaktschicht wird ein aus 2-Ethylhexylacrylat, Vinylacetat und 2-Hydroxyethylacrylat hergestellter Polyacrylatkleber verwendet. Zu 31,87 Kg einer Lösung dieses Klebers mit einem Feststoffgehalt von 51 Gew.-% gibt man 6,5 kg Ethylacetat und 1,91 Kg Ölsäure in Reinform oder als Gemisch mit anderen Carbonsäuren und erhält nach Homogenisierung ca. 40 Kg wirkstofffreie Polyacrylatlösung (Kleberlösung 2).
C Mit dem Fachmann bekannten Hilfsmitteln beschichtet man eine für den gewählten Kleber abhäsiv ausgerüstete Folie mit der buprenorphinhaltigen Kleberlösung 1. Die Beschichtungsdicke wird so gewählt, dass nach Entfernen der Lösemittel ein Beschichtungsgewicht der Matrixschicht von 55 g/m² resultiert. Die Konzentration von Buprenorphin und Lävulinsäure in dieser Schicht beträgt 7 bis 9 Gew.-%. Auf die "getrocknete" Matrixschicht wird dann die Rückschicht des späteren Systems laminiert.
   Kleberlösung 2 wird ebenfalls auf eine abhäsiv ausgerüstete Folie (spätere vor Gebrauch der Systeme zu entfernende Schutzfolie) beschichtet und die organischen Lösemittel entfernt. Die Beschichtungsdicke der resultierenden Hautkontaktschicht soll nach Entfernung der Lösemittel ca. 20 g/m² betragen. Von der zuerst hergestellten Matrixschicht wird nun die abhäsiv ausgerüstete Folie entfernt und die Matrixschicht auf die Hautkontaktschicht kaschiert. Aus dem resultierenden Gesamtlaminat können nun die einzelnen Systeme gestanzt werden.

In speziellen Ausführungsformen kann ein TTS, wie oben beschrieben, mit einem flächenmäßig größeren Überpflaster, vorzugsweise mit gerundeten Ecken, versehen werden, umfassend eine haftklebende wirkstofffreie Matrixschicht mit bevorzugt hautfarbener Rückschicht. Dies ist von Vorteil, wenn die Hautkontaktschicht auf Grund ihrer physikalischen Eigenschaften alleine nicht ausreichend auf der Haut klebt und/oder wenn die buprenorphinhaltige Matrixschicht zur Vermeidung von Abfall ausgeprägte Ecken aufweist (z.B. quadratische oder rechteckigen Formen).

### Beispiel 2 - 5

Die Herstellung erfolgt analog Beispiel 1, lediglich die Konzentrationen und Schichtdicke der Matrixschicht wurden gemäß Tabelle 1 variiert.

### Beispiel 6

Als Beispiel 6 wurde das Handelsprodukt Transtec® der Grünenthal GmbH verwendet.

**Tabelle 1: Zusammensetzung des buprenorphinhaltigen TTS bezogen auf die wirkstoffllaltige Matrix**

| Beispiel | Flächengewicht Matrixschicht | Buprenorphingehalt in der Matrixschicht | Buprenorphingehalt | Lävulinsäuregehalt Laminat 1 |
|---|---|---|---|---|
| | [g/m²] | [Gew.-%] | [mg/cm²] | [Gew.-%] |
| 1 | 55 | 7 | 0,385 | 7 |
| 2 | 60 | 7 | 0,42 | 7 |
| 3 | 65 | 8,4 | 0,546 | 8,4 |
| 4 | 80 | 7 | 0,56 | 7 |

Mit diesen TTS wurden in vitro Versuche mit der dem Fachmann bekannten Diffusionszelle nach Franz unter Verwendung von Epidermis aus Human-Vollhaut durchgeführt. Hierzu wurden jeweils Stanzlinge mit einer Fläche von 2,54 cm² aus Laminaten gestanzt und gegen Stanzlinge des Handelsproduktes Transtec® getestet. Transtec® ist im Handel in drei unterschiedlichen Dosisstärken erhältlich; sie sind aber flächenproportional. Es wurden die Konzentrationen von Buprenorphin im Akzeptormedium der Franz-Zelle gemessen (Tab. 2). Zusätzlich wurden die TTS nach dem Experiment auf ihren Gehalt an Buprenorphin und Lävulinsäure analysiert. Die Ergebnisse der Analysen von Beispiel 1 sind tabellarisch und graphisch neben denen der weiteren Beispiele dargestellt.

**Tabelle 2: Mittlere kumulierte Mengen an Buprenorphin in Mikrogramm/Stunde, die an der Franz-Zelle aus den erfindungsgemäßen TTS freigesetzt wurden.**

| Beispiel | 2 h | 4 h | 8 h | 24 h | 32 h | 48 h | 56 h | 72 h |
|---|---|---|---|---|---|---|---|---|
| 1 | <NG | 0,015 | 0,118 | 1,79 | 3,40 | 7,56 | 13,6 | 21,1 |
| 2 | <NG | 0,007 | 0,062 | 0,87 | 1,72 | 5,3 | 9,63 | 19,3 |
| 3 | 0,013 | 0,027 | 0,076 | 0,689 | 1,36 | 4,7 | 9,15 | 21,5 |
| 4 | 0,035 | 0,071 | 0,184 | 1,64 | 3,27 | 8,86 | 12,9 | 25,9 |
| Transtec® | n.b. | 0,061 | 0,167 | 2,35 | n.b. | 11,4 | n.b. | 25,4 |

Vergleicht man die kumulierten Flux-Raten aus Tabelle 2 untereinander, so zeigt sich, dass alle Permeationsraten der TTS gemäß der Erfindung in der Größenordnung des Handelspräparats Transtec® liegen. Auch wenn die Franz-Zelle nicht als Ersatz für klinische Prüfungen dient, sondern zur Diskriminierung zwischen verschiedenen TTS-Rezepturen benutzt wird, können die in Tabelle 2 dargestellten Ergebnisse dahingehend bewertet werden, dass unter in vitro Bedingungen TTS nach Beispiel 1 ebenso viel Buprenorphin abgibt wie Transtec®. Wie vorangehend bereits beschrieben, wurde ein TTS gemäß Beispiel 1 in einer Pharmakokinetikstudie am Menschen mit diesem bereits vermarkteten TTS als Referenzsystem verglichen und für das Referenzprodukt eine Wirkstoffausnutzung von 17 % TTS gegenüber einer Wirkstoffausnutzung von 53 % eines TTS gemäß Beispiel 1 nachgewiesen.

Es wurden nach den Permeationsstudien alle erfindungsgemäßen Beispiel TTS auf ihre restlichen Lävulinsäuregehalte untersucht. Die Restmengen und die hieraus errechneten relativ abgegebenen Mengen an Lävulinsäure sind in Tabelle 3 wiedergegeben.

**Tabelle 3: Freisetzung von Lävulinsäure**

| Beispiel | Lävulinsäuregehalt [mg/cm²] | Rest Lävulinsäure im TTS [mg/cm²] | Freigesetzte Lävulinsäure [%] |
|---|---|---|---|
| 1 | 0,385 | 0,025 | 93,5 |
| 2 | 0,42 | 0,026 | 93,8 |
| 3 | 0,546 | 0,033 | 94 |
| 4 | 0,56 | 0,039 | 93 |

Tabelle 3 verdeutlicht, dass gemäß der erfindungsgemäßen Lehre die TTS während der Anwendung an Lävulinsäure verarmen und damit die überraschend hohe Wirkstoffausnutzung an Buprenorphin bedingen.

## Patentansprüche

1. Transdermales therapeutisches System zur Verabreichung von Buprenorphin an die Haut umfassend eine wirkstoffundurchlässige Rückschicht, mindestens eine haftklebende, den Wirkstoff Buprenorphin und mindestens eine Carbonsäure enthaltende Matrixschicht und gegebenenfalls eine vor Gebrauch ablösbare Schutzschicht, **dadurch gekennzeichnet, dass** die Matrixschicht auf Basis von Polysiloxanen oder Polyisobutylen aufgebaut ist, das Buprenorphin in der Carbonsäure oder den Carbonsäuren gelöst und diese Lösung in Form von Tröpfchen in der Matrixschicht dispergiert ist.

2. Transdermales therapeutisches System nach Anspruch 1, **dadurch gekennzeichnet, dass** das Polysiloxan ein aminresistentes Dimethylpolysiloxan ist.

3. Transdermales therapeutisches System nach Anspruch 1, **dadurch gekennzeichnet, dass** das Polysiloxan ein Gemisch aus einem aminresistenten und einem nicht-aminresistenten Dimethylpolysiloxan ist, worin das nicht-aminresistente Dimethylpolysiloxan bis zu 40 Gew.-% enthalten ist.

4. Transdermales therapeutisches System nach einem oder mehreren der vorangehenden Ansprüchen, **dadurch gekennzeichnet, dass** die Carbonsäure schneller als der Wirkstoff Buprenorphin in die Haut diffundiert.

5. Transdermales therapeutisches System nach einem oder mehreren der vorangehenden Ansprüchen, **dadurch gekennzeichnet, dass** die Menge der dispergierten Lösung bis zu 40 Gew.-%, bevorzugt bis zu 20 Gew.-% beträgt.

6. Transdermales therapeutisches System nach einem oder mehreren der vorangehenden Ansprüchen, **dadurch gekennzeichnet, dass** die Carbonsäure bei Hauttemperatur flüssig ist.

7. Transdermales therapeutisches System nach Anspruch 6, **dadurch gekennzeichnet, dass** die Carbonsäure ausgewählt ist aus der Gruppe bestehend aus Ölsäure, Lävulinsäure, Linolsäure, und Linolensäure.

8. Transdermales therapeutisches System nach Anspruch 6 oder 7, **dadurch gekennzeichnet, dass** Buprenorphin und Lävulinsäure im gleichen Gewichtsverhältnis vorliegen.

9. Transdermales therapeutisches System nach einem oder mehreren der vorangehenden Ansprüche, **dadurch gekennzeichnet, dass** eine Wirkstoffausnutzung unter in vivo Bedingungen von mindestens 30 %, bevorzugt mindestens 40 % und besonders bevorzugt mindestens 50 % erreicht wird.

10. Transdermalen therapeutischen Systems nach einem oder mehreren der vorangehenden Ansprüche zur Verwendung in der Schmerztherapie.

## Claims

1. Transdermal therapeutic system for administering buprenorphine to the skin, comprising an active ingredient-impermeable backing layer, at least one pressure-sensitive adhesive matrix layer comprising the active ingredient buprenoiphine and at least one carboxylic acid, and, optionally, a protective layer to be detached before use, **characterized in that** the matrix layer is constructed on the basis of polysiloxanes or polyisobutylene, the buprenorphine is in solution in the carboxylic acid or carboxylic acids, and this solution is in dispersion in the form of droplets in the matrix layer.

2. Transdermal therapeutic system according to claim 1, **characterized in that** the polysiloxane is an amine-resistant dimethylpolysiloxane.

3. Transdermal therapeutic system according to claim 1, **characterized in that** the polysiloxane consists of a mixture of an amine-resistant and a non-amine-resistant dimethylpolysiloxane, where the non-amine-resistant dimethylpolysiloxane is present at up to 40% by weight.

4. Transdermal therapeutic system according to one or more of the preceding claims, **characterized in that** the carboxylic acid diffuses into the skin more quickly than does the active ingredient buprenorphine.

5. Transdermal therapeutic system according to one or more of the preceding claims, **characterized in that** the amount of the dispersed solution is up to 40% by weight, preferably up to 20% by weight.

6. Transdermal therapeutic system according to one or more of the preceding claims, **characterized in that** the carboxylic acid is liquid at skin temperature.

7. Transdermal therapeutic system according to claim 6, **characterized in that** the carboxylic acid is selected from the group consisting of oleic acid, laevulinic acid, linoleic acid and linolenic acid.

8. Transdermal therapeutic system according to claim 6 or 7, **characterized in that** buprenorphine and laevulinic acid are present in the same weight ratio.

9. Transdermal therapeutic system according to one or more of the preceding claims, **characterized in that** an active substance utilization under in vivo conditions of at least 30%, preferably at least 40% and more preferably at least 50% is achieved.

10. Transdermal therapeutic system according to one or more of the preceding claims for use in pain therapy.

## Revendications

1. Système thérapeutique transdermique pour l'administration de buprénorphine sur la peau comprenant une couche arrière imperméable à la substance active, au moins une couche matrice contenant la substance active buprénorphine et au moins un acide carboxylique et le cas échéant une couche protectrice amovible avant usage, **caractérisé en ce que** la couche matrice est à base de polysiloxanes ou de polyisobutylène, la buprénorphine est dissoute dans l'acide carboxylique ou les acides carboxyliques et cette solution est dispersée sous forme de gouttelettes dans la couche matrice.

2. Système thérapeutique transdermique selon la revendication 1, **caractérisé en ce que** le polysiloxane est un diméthylpolysiloxane résistant aux amines.

3. Système thérapeutique transdermique selon la revendication 1, **caractérisé en ce que** le polysiloxane est un mélange constitué d'un diméthylpolysiloxane résistant aux amines et d'un autre non résistant aux amines, où le diméthylpolysiloxane non résistant aux amines est présent jusqu'à un taux de 40% en poids.

4. Système thérapeutique transdermique selon l'une ou plusieurs des revendications précédentes, **caractérisé en ce que** l'acide carboxylique se diffuse plus rapidement dans la peau que la substance active buprénorphine.

5. Système thérapeutique transdermique selon l'une ou plusieurs des revendications précédentes, **caractérisé en ce que** la quantité de solution dispersée atteint jusqu'à 40% en poids, de préférence jusqu'à 20% en poids.

6. Système thérapeutique transdermique selon l'une ou plusieurs des revendications précédentes, **caractérisé en ce que** l'acide carboxylique est liquide à la température de la peau.

7. Système thérapeutique transdermique selon la revendication 6, **caractérisé en ce que** l'acide carboxylique est choisi dans le groupe constitué par l'acide oléique, l'acide lévulinique, l'acide linolique et l'acide linolénique.

8. Système thérapeutique transdermique selon la revendication 6 ou 7, **caractérisé en ce que** la buprénorphine et l'acide lévulinique sont présents en le même rapport en poids.

9. Système thérapeutique transdermique selon l'une ou plusieurs des revendications précédentes, **caractérisé en ce que** l'on atteint une utilisation de la substance active dans des conditions in vivo d'au moins 30%, de préférence d'au moins 40% et particulièrement préférentiellement d'au moins 50%.

10. Système thérapeutique transdermique selon l'une ou plusieurs des revendications précédentes pour une utilisation dans le traitement de la douleur.
